# EUROPEAN PATENT SPECIFICATION

(11) **EP 2 041 288 B1**
(45) Date of publication and mention of the grant of the patent: **02.03.2011**
(21) Application number: 07733132.0
(22) Date of filing: 08.06.2007
(51) Int. Cl.: C12N 15/70

(54) **RK2-BASED BROAD-HOST-RANGE CLONING VECTOR USEFUL FOR TRANSFER OF METAGENOMIC LIBRARIES TO A VARIETY OF BACTERIAL SPECIES**
KLONIERUNGSVEKTOR MIT BREITBANDWIRTSBEREICH AUF RK2-BASIS MIT EIGNUNG ZUR ÜBERTRAGUNG METAGENOMISCHER BIBLIOTHEKEN IN VERSCHIEDENE BAKTERIENSPEZIES
VECTEUR DE CLONAGE A BASE PLASMIDIQUE RK2 ET LARGE SPECTRE D'HOTES POUR LE TRANSFERT DE BIBLIOTHEQUES METAGENOMIQUES A UNE GRANDE VARIETE D'ESPECES BACTERIENNES

(30) Priority: 09.06.2006 GB 0611484
(43) Date of publication of application: 01.04.2009
(73) Proprietor: Sinvent AS, 7465 Trondheim (NO)
(72) Inventor: AAKVIK, Trine, 7026 Trondheim (NO); DEGNES, Kristin, Fløgstad, 7051 Trondheim (NO); ELLINGSEN, Trond, Erling, 7054 Ranheim (NO); SKREDE, Rannveig, 7048 Trondheim (NO); VALLA, Svein, 7560 Trondheim (NO)
(74) Representative: Dzieglewska, Hanna Eva
(86) International application number: PCT/GB2007/002122
(87) International publication number: WO 2007/141540

(56) References cited:
- WO-A-2004/033633
- TAO Q ET AL: "CLONING AND STABLE MAINTENANCE OF DNA FRAGMENTS OVER 300 KB IN ESCHERICHIA COLI WITH CONVENTIONAL PLASMID-BASED VECTORS" NUCLEIC ACIDS RESEARCH, OXFORD UNIVERSITY PRESS, SURREY, GB, vol. 26, no. 21, 1998, pages 4901-4909, XP002933437 ISSN: 0305-1048
- WILLIAMSON LYNN L ET AL: "Intracellular screen to identify metagenomic clones that induce or inhibit a quorum-sensing biosensor." APPLIED AND ENVIRONMENTAL MICROBIOLOGY OCT 2005, vol. 71, no. 10, October 2005 (2005-10), pages 6335-6344, XP002463964 ISSN: 0099-2240 -& WILD JADWIGA ET AL: "Conditionally amplifiable BACs: switching from single-copy to high-copy vectors and genomic clones." GENOME RESEARCH SEP 2002, vol. 2512, no. 9, September 2002 (2002-09), pages 1434-1444, XP002463965 ISSN: 1088-9051
- BLATNY J M ET AL: "Construction and use of a versatile set of broad-host-range cloning and expression vectors based on the RK2 replicon." APPLIED AND ENVIRONMENTAL MICROBIOLOGY FEB 1997, vol. 63, no. 2, February 1997 (1997-02), pages 370-379, XP002047393 ISSN: 0099-2240
- EASTER CARLA L ET AL: "Contribution of different segments of the par region to stable maintenance of the broad-host-range plasmid RK2" JOURNAL OF BACTERIOLOGY, WASHINGTON, DC, US, vol. 179, no. 20, 1997, pages 6472-6479, XP002191826 ISSN: 0021-9193
- DITTA G ET AL: "Broad host range DNA cloning system for gram-negative bacteria: construction of a gene bank of Rhizobium meliloti." PROCEEDINGS OF THE NATIONAL ACADEMY OF SCIENCES OF THE UNITED STATES OF AMERICA DEC 1980, vol. 77, no. 12, December 1980 (1980-12), pages 7347-7351, XP002035714 ISSN: 0027-8424

## Description

The present invention relates to a new cloning vector for use in metagenomic studies which allows the transfer of metagenomic libraries to a wide range of bacterial hosts. The vector is based on the RK2 plasmid and includes in particular the RK2 origins of replication and transfer, *oriV* and *oriT.* The invention provides for the first time, and unexpectedly, a vector capable of maintaining large-sized inserts (as well as smaller ones) at increased eg. medium to high copy number in a broad range of hosts.

Natural environments,such as soil or water, contain vast numbers of microorganisms. The majority cannot be cultivated, and therefore evade conventional analysis of their genomes. An enormous source of genetic information therefore remains undiscovered even after extensive screening based on standard cultivation methods. This difficulty can be avoided by studying the "metagenome" of the environmental sample, that is the collective genomes of all the microrganisms within it. The metagenome has spatial and temporal dimensions; environments change and a sample relates to a particular time and place. Metagenomics seeks to discover the identity of genes within environments and to demonstrate their functions and interactions. Many thousands of uncultured microrganisms have been identified by metagenomic studies and novel genes have been discovered, including in particular on the basis of their function, rather than their sequence.

More particularly, in the metagenomic approach, such environmental sources may be explored by direct isolation and cloning of DNA from environmental samples into suitable vectors, thus creating complex metagenomic libraries. Different vectors, including plasmids with small inserts and cosmids, fosmids and BACs (bacterial artificial chromosomes) have been used for making metagenomic libraries for functional screening, or using bacteriophage λ, to detect novel polypeptides. Hitherto, a number of metagenomic libraries have been made from both soil and marine environments (reviewed in Daniel, 2005, Nature Rev 3: 470-478; DeLong, 2005, Nature Rev 3: 459-469). In addition, Venter and colleagues reported the first example of the use of the "whole-genome shotgun sequencing" approach to marine microbial populations collected from the Sargasso Sea (Venter et al., (2004), Science 304: 66-74).

Metagenomic libraries can be analyzed for novel genes and pathways with sequence-based techniques or through activity screening involving analyses of expression of novel phenotypic traits in surrogate hosts.

Mostly metagenomic vectors replicate only in *Escherichia coli* or its close relatives. The vectors most often used for large insert metagenomic studies are the *Escherichia coli* F-factor based vectors for BAC (bacterial artificial chromosome) and fosmid (packing by λ-phage) cloning (Shizuya et al., 1992, Proc Natl acad Sci, USA 89: 8794-8797). The replication of such vectors and therefore the expression of the metagenome libraries, are limited to the use of *E. coli* strains as hosts. Identification of novel activities is dependent on successful transcription and translation of the cloned genes, and although novel activities have been expressed using *E. coli* as host, there is a potential advantage of expanding the range of bacterial hosts to capture additional expression capability. This was recently demonstrated by Gabor *et al.* (Gabor et al., 2004, Environ Microbiol 6: 879-886) in a study showing that only 40% of the genes from the genomes of 32 prokaryotes could be detected when expressed in *E. coli.* The study also revealed significant differences in the predicted expression modes between distinct taxonomic groups of organisms. Another study by Martinez and co-workers showed that *E. coli, Pseudomonas putida* and *Streptomyces lividans* differed in their abilities to express heterologous gene clusters (Martinez et al., 2004, Appl. Environ Microbiol 70: 2452-2463). '

To exploit the potential involved in using many different hosts for expression of genes obtained from environmental samples new biological tools are needed. Some shuttle vectors with a host range wider than the *E.coli* metagenomic vectors discussed above have been reported: For example, BAC vectors that are capable of being transferred to one or two hosts in addition to *E. coli* (Sosio et al., 2000, Nature Biotechnol 18: 343-345; Martinez et al., 2004, Appl. Environ Microbiol 70: 2452-2463) have been described. However, these vectors are based on integration of the environmental DNA into the host chromosome.

WO 2004/033633 discloses broad-host-range vectors for the cloning of environmental DNA probes. In particular, RK2 based fosmids are disclosed in which larger fragments (> 40 kb) of such DNA can be cloned.

So far, small autonomously replicating vectors capable of carrying very large inserts and suitable for use in a broad-host-range, have not been developed for metagenomic studies.

The present invention has been developed to meet this need. In particular, novel, relatively small and functionally well understood vectors have been developed, based on the broad-host-range RK2 replicon. Such vectors have a utility in the construction of metagenomic libraries. It is generally known that vectors constructed from this replicon function in numerous Gram-negative bacterial species (Thomas & Helinski, 1989 Promiscuous Plasmids in Gram-negative bacteria (Thomas, C.M., Ed.) Chapter 1, pp 1-25, Academic Press Inc (London) Ltd, London), and have even been transferred to Gram-positive bacteria, yeast and mammalian cells (Poyart and Trieu-Cout, 1997, FEMS Microbiol Lett 156: 193-198; Bates et al., 1998, J Bacteriol 180: 6538-6543; Waters, 2001, Nature Genetics 29: 375-376). However, it has not previously been appreciated or thought possible that vectors such as now proposed, and in particular such vectors which do not integrate into the host chromosome, could be used to clone, stably maintain and express large inserts in a broad range of species, and hence could be used as the basis for transferable vectors for metagenomic use. The capability of the vector of the invention to replicate in many hosts means that entire libraries can be transferred from *E. coli* to a number of hosts, and this is efficiently achieved by conjugation. RK2 replicons display increased copy number (ie. a copy number greater than 1, typically about 5-10), and the gene dosage of cloned inserts will be significantly higher than for chromosomal insertions, enhancing the chances that functional assays will succeed. In addition it is possible further to increase the copy-number across species barriers through the use of copy-up mutants of the essential replication initiation gene *trfA* (Haugan et al., 1995, Plasmid 33: 27-39). It is an unexpected and surprising feature of the present invention that large inserts (eg. of 30 or 40 kb or more, or even 50, 60, 80 or 100 kb or more) may be cloned and maintained in a relatively high copy number vector (plasmid) in a broad range of bacterial species (ie. across a broad host range). The present invention enables transfer of such vectors (plasmids) containing large inserts to a broad range of bacteria, and the stable maintenance of such vectors (plasmids) in the broad host range.

The present invention accordingly provides a cloning vector for cloning of DNA in a broad host range of bacteria, said vector being an autonomously replicating artificial chromosome comprising:
(i) the RK2 origin of replication *oriV*;
(ii) the RK2 origin of conjugative transfer *oriT*;
(iii) *parDE* from RK2
(iv) a cloning region;
(v) a further origin of replication which permits replication of said vector at a copy number of no more than 1 or 2;
   wherein said vector is no more than 15kb, in size, does not contain *trfA* of RK2, and is capable of cloning inserts of at least 12kb and wherein the content of RK2 DNA in said vector is no more than 10% of RK2.
   RK2 as referred to above is the RK2 plasmid discussed further below (see Thomas and Helinski, 1989 supra).

More particularly, the vector is capable of cloning inserts of at least 15, 20, 30 or 40kb. Accordingly, alternatively put, the vector is capable of cloning large inserts. "Large inserts" are defined herein as inserts at least 30kb in size, more particularly of at least 40, 50, 60 or 70kb. In a particularly advantageous embodiment of the invention inserts of at least 80kb may be cloned, more particularly at least 90 or 100kb. Advantageously, inserts of 120, 150, 170, 180, 190 or 200kb or more may also be cloned. Alternatively put, inserts of up to 100, 120, 150, 170, 190, 200 or 250 kb can be cloned, for example such inserts of at least 12, 15, 20 or 30, 50, 70 or 80 kb up to any of the aforementioned figures. More particularly, the vector of the invention allows such large inserts to be stably maintained. The vectors containing the large inserts may be stably maintained in a broad host range. Further the vectors containing such large inserts are capable of being transferred to a broad host range.

The vector of the invention is suitable for, and may be used for, metagenome cloning. It may accordingly be referred to as a metagenomic cloning vector.

The vector of the present invention permits the cloning of DNA, advantageously, as stated above, the cloning of metagenomic DNA. The DNA may thus be genomic DNA, but in the broadest concept of the invention it may be any DNA. The DNA may be from any source. Thus, genomic DNA, cDNA or any type of synthetic DNA is included, for example cloned or amplified DNA fragments. The DNA may be from a single source or a mixture of sources eg. from a single sample or a mixture of samples. It may represent a single DNA or a mixture of DNAs eg. from a single organism or from a mixture of organisms (ie. at least 2 organisms). Preferably however, the DNA is metagenomic DNA or DNA from an environmental sample. The DNA may thus be isolated or obtained from an environmental sample.

A particular advantage of the present invention is the broad host range of the vector. This means that the vector may be used in wide range of different bacterial genera or species eg. in at least 5, 7, 10 or 12 different bacteria, eg. at least 5 unrelated bacteria. The host range of the RK2 plasmid and RK2-based or derived plasmids or replicons in general is discussed in Thomas and Helinski, 1989 (supra) and all such bacteria may be used according to the present invention. The broad host range of the vectors thus includes a vast range of Gram-negative bacteria, as well as Gram-positive bacteria. Suitable Gram-negative bacteria include all enteric species, including, for example, *Escherichia* sp., *Salmonella, Klebsiella, Proteus* and *Yersinia.* and non-enteric bacteria including *Azotobacter* sp., *Pseudomonas* sp., *Xanthomonas* sp., *Caulobacter* sp, *Acinetobacter* sp., *Aeromonas* sp., *Agrobacterium* sp., *Alcaligenes* sp., *Bordatella* sp., *Haemophilus Influenzae, Methylophilus methylotrophus, Rhizobium* sp. and *Thiobacillus* sp. (see also Thomas and Helinski, supra). Gram-positive bacterial hosts which may be used include *Clavibacter* sp. More particularly, the vector may be used in a large range of or nearly all gram negative bacteria. Representative gram negative genera include Pseudomonas, Xanthomonas and enteric bacteria, although this list is non-exhaustive and the hosts selected may depend on the study, sample selected, etc. Although gram negative hosts are preferred, the vectors of the invention may also be used in gram positive bacteria, to which RK2-based plasmids are known to be transferable eg. *Clavibacter sp.*

Such transformed host cells are included within the scope of the present invention. A further aspect of the present invention thus includes a host cell containing an cloning vector as hereinbefore defined.

The vector is capable of autonomous replication. This means that it is an non-integrating vector ie. it does not integrate into the host chromosome. In particular it is non-integrating in any host into which it is introduced or to which it it may be transferred. The vector is capable of self-replication in a bacterial host, such that the vector remains present (as a vector) when the bacterial cell grows and divides. More particularly, the vector is capable of being stably maintained in its bacterial host. Thus, the vector may be introduced into a bacterial host and may be maintained (ie. the presence of the vector may be detected) in that host during culture of that host over repeated generations (e.g. over at least 2, 3, 4, 5, 6 or 10 generations) or more generally during growth of the host cells.

Autonomous replication of course requires that the host is capable of supporting replication of that vector ie. that the host contains the necessary genetic machinery. An appropriate host may be selected or genetically engineered and this is within the routine skill of a person skilled in the art. Thus, for example although the vector contains two origins, replication across the broad host range will generally be from *oriV.* This means that the host will require the *trfA* gene for replication to occur, as explained further below, and as discussed below, this may readily be introduced into the host, particularly into the host chromosome.

The term "artificial chromosome" is used herein to include any artificially constructed self-replicating genetic element which is capable of carrying a large insert (e.g. an insert of 30 kb or more, or larger inserts as discussed above). The artificial chromosome is thus a genetic construct capable of autonomous replication in a bacterial host and capable of carrying a large insert. The construct is capable of behaving or functioning as a chromosome in the bacterial host. The artificial chromosome is accordingly capable of being stably maintained in a bacterial host cell. The artificial chromosome includes an origin of replication and any other genetic elements or sequences needed for it to propagate from one bacterial cell to its offspring.

Accordingly, at its broadest the cloning vector of the present invention may be seen as a plasmid, or plasmid-type vector, capable of autonomous replication in a host cell, which can stably carry a large insert.

The artificial chromosome is preferably a bacterial artificial chromosome (BAC). BACs or BAC vectors are well known in the art and widely described in the literature. In a preferred embodiment, a BAC may broadly be defined as a DNA construct, based on a fertility plasmid, used for transforming and cloning in bacteria. BACs typically have a large insert size range eg. 100 to 300kb. In practice, BAC vectors are typically modified plasmids that contain an origin of replication from the the *E.coli* F-factor (commonly referred to as *ori2* or *oriS*) (Shizuya et al. 1992, PNAS 89, 8794-8797). The full F-factor sequence is available under NCIB accession number AP001918. The F-factor origin controls replication strictly at one or two copies per cell. As a BAC vector, the cloning vector of the invention may accordingly contain the F origin of replication, or *ori2.* Accordingly, when the vector of the invention is a BAC vector, the further origin of replication of feature (v) above may be *ori2.* More particularly, a BAC vector of the invention may contain the F-factor replicon. This is defined as *ori2, repE* and *parAB.* Sources and sequences of these genetic elements are readily available in the art. Thus for example, *ori2* may be obtained from the F-factor or from any other plasmid or vector which is based on the F-factor or which contains *ori2*. *parAB* may be obtained from any appropriate source eg. the F-factor or any F-factor based or derived plasmid or vector or indeed any vector or plasmid which contains *parAB* genes, which need not necessarily be derived from the F-factor. P1 for example also has *parAB* genes which could be used. The pCC1 BAC and fosmid vectors from Epicentre Technologies have *ori2* and *parAB* which could be used. *ori2* and *repE* mediate unidirectional replication of the F factor while *parA* and *parB* maintain copy number at one or two per *E. coli* genome. The F replicon may additionally include the further stabilisation function *parC* and/or the *redF* gene. Thus, in a preferred embodiment of the invention, the vector of the invention is a BAC vector comprising *ori2, repE, and parAB,* more particularly a BAC vector comprising *ori2, repE,* and *parABC* and optionally *redF.*

In a further embodiment, the vector of the invention may be based on a P1-derived artificial chromosome, PAC (Ioannou et al. 1994 Nat Genet 6, 84-89; Sternberg 1990 PNAS 87, 103-107), which may be viewed as a sub-set of BACs. A PAC is based on and hence comprises the bacteriophage P1 origin of replication. Accordingly, when the vector of the invention is a PAC vector, the further origin of replication of feature (v) above maybe the P1 origin of replication. More particularly, the PAC vector contains a P1 plasmid replicon. A PAC vector may, if desired, further comprise a P1 packaging site (pac) to package vector and cloned DNA into phage particles, and two P1 *loxP* recombination sites to cyclize the packaged DNA (when in a an appropriate E. coli host containing the P1 Cre recombinase). Again, sources and sequences of such genetic elements are readily available and described in the literature.

The further, or second, origin of replication, permits replication of the vector at low copy number of no more than 1 or 2. As noted above, this may be *ori2* or the P1 origin of replication. It may however be any origin which permits replication of the vector in the initial host of choice at low copy number. As explained further below, the vector of the invention may generally be used first to establish a library of cloned inserts in a selected host. The library may then be transferred from that initial or first host to one or more different hosts, preferably a broad range of hosts. The RK2 origin *oriV* is a broad host range origin and confers the property of broad host range on the vector. *oriV,* however, requires *trfA* and since this is absent from the vector, this must be separately provided to the host, if not present naturally. Thus in the absence of *trfA,* replication will only occur from the second or further origin. This origin may therefore be selected to function in the host selected for initial construction of the library. Since *E. coli* will generally be a preferred such initial host, it is preferred that the further origin be functional in *E. coli.* Since vectors expressed at low copy number may be more stable, the low copy number of this origin may be advantageous in the practice of the invention, where the first or initial host functions as a "storage depot" for the cloned DNA ie. the DNA may initially be cloned at low copy number in the first host to which the vector is introduced, before being transferred at higher (eg. medium) copy number to other hosts.

The vector of the invention contains a number of functions or features of the RK2 plasmid, most notably *oriV, oriT* and *parDE.* RK2 is a well-characterised naturally occurring 60Kb self-transmissible plasmid of the IncP incompatibility group well known for its ability to replicate in a wide range of gram-negative bacteria (Thomas and Helinski, 1989, in Promiscuous Plasmids in Gram-negative bacteria (Thomas, C.M., Ed.) Chapter 1, pp 1-25, Academic Press Inc (London) Ltd, London). It has been determined that the minimal replicating unit of RK2 consists of two genetic elements, the origin of vegetative replication (*oriV*), and a gene (*trfA*) encoding an essential initiator protein (TrfA) that binds to short repeated sequences (iterons) in *oriV* (Schmidhauser and Helinski, 1985, J. Bacteriol. 164, 446-455; Perri et al., 1991, J. Biol. Chem; 266, 12536-12543). This minimal replicating unit is termed the so-called "RK2 minimum replicon", and has been extensively characterised and studied in the literature. A wide range of replicons (termed "mini-RK2 replicons") and cloning vectors based on the RK2 minimum replicon or on derivatives of the RK2 plasmid have been prepared and described in the literature (see, for example, Li et al., 1995, J. Bacteriol. 177, 6866-6873; Morris et al., J. Bacteriol., 177, 6825-6831; Franklin and Spooner, in Promiscuous Plasmids in Gram-negative bacteria (Thomas, C.M., ed) Ch. 10, pp 247-267, Academic Press Inc. (London) Ltd., London; Haugan et al., 1992, J. Bacteriol 174:7026-7032; and Valla et al., 1991, Plasmid, 25,131-136; Blatny et al., 1997, Appl Environ Microbial 63: 370-379; Blatny et al., 1997, Plasmid 38: 35-51; Santos et al., 2001 FEMS Microbiol.. Lett. 195: 91-96). Further, the complete nucleotide sequence of RK2 is reported (Pansegrau et al., 1994, J. Mol. Biol., 239, 623-633). The *parDE* genes are part of an operon in RK2 which is involved in the maintenance of the plasmid or heterologous replicons in diverse bacterial hosts (Roberts et al., 1990, J. Bacteriol, 172, 6204-6216; Schmidhauser and Helinski, supra; Sia et al., 1995, J. Bacteriol, 117, 2789-2797; Roberts et al., 1992, J. Bacteriol, 174, 8119-8132). Thus, sources for the RK2 minimum replicon and other RK2 elements are well established and readily available. Hence, for example, the RK2 *oriV, oriT* or *parDE* may be derived from the parental plasmid RK2 or from any of the vast number of derivatives or mini RK2 plasmids described and available from the literature (see e.g. Li et al; Morris et al., Franklin and Spooner; Haugen et al; and Valla et al., Blatny et al., supra). BAC and fosmid vectors containing the RK2 *oriV* are also available (eg. pCC1BAC and pCC1FOS from Epicentre) and these may be used as starting or source plasmids, as described further in Example 1 below. The separate RK2 elements may be isolated from the same source together or separately or from separate sources.

Techniques for excising the desired nucleotide sequences containing the required RK2 elements or functions from a selected source and introducing them into a vector or intermediate construct are well known and standard in the art, and are described for example in Sambrook et al., 1989, Molecular cloning; a laboratory manual, 2nd Edition, Cold Spring Harbour Laboratory Press, Cold Spring Harbour, N.Y.

As will be described in more detail in the Examples below, it is convenient to isolate the desired sequences from a selected source and introduce them, using techniques standard in the art, into a series of intermediate constructs, which may be plasmids, introducing or adding or deleting elements to arrive at the vectors of the invention. Alternatively a starting plasmid or vector may be selected, already containing some of the desired elements (eg. pCC1FOS as described in Example 1 below), and this may be modified to introduce the further features of the vectors of the invention (for example pCC1FOS may be modified by the introduction of *oriT* and *parDE*).

As used herein the terms *"oriV", "trfA" "oriT", "parD", "parE", "ori2" "paiA", "parB", parC", "repE", "redF"* etc and any other named gene or genetic element include not only the native or wild-type functions as they appear in the original, parental or archetypal source plasmids but also any modifications of the functions, for example by nucleotide addition, deletion, or substitution or indeed chemical modification of the nucleotides, which occur naturally, e.g. by allelic variation or spontaneous mutagenesis, or which are introduced synthetically. Techniques for modification of nucleotide sequences are standard and well known in the literature and include for example mutagenesis, e.g. the use of mutagenic agents or site-directed mutagenesis. PCR may also be used to introduce mutations. Appropriate or desired mutations may for example be selected by mutant screening of the genetic element in question.

The vectors of the invention further contain a cloning region (or alternatively put, a cloning segment). This includes in particular a site for introduction of the DNA to be cloned (ie. the "insert"). The cloning region thus includes a cloning site. Conveniently such a cloning site may be or may comprise one or more restriction sites. Multiple, e.g. at least 2 or 3, up to 20 or more, such insertion sites may be contained. Vectors containing multiple restriction sites may be constructed, containing eg. 2-20, 3-20, 2-10, 3-10, 3-6, or 2-6 unique sites eg. in a polylinker. Suitable cloning sites for insertion of a desired gene are well known in the art and widely described in the literature, as are techniques for their construction and/or introduction into the vectors of the invention (see eg. Sambrook et al., supra).

For ease of construction, appropriate cloning sites may be introduced in the form of a polylinker sequence, using nucleic acid manipulation techniques which are standard in the art. A range of suitable polylinker sequences are known in the art and may simplify the routine use of the vectors. Thus, for example a well-known polylinker/*lacZ*' region may be used, as described for example in the vectors of Ditta et al., 1985, Plasmid, 13, 149-153, simplifying standard cloning procedures and identification of plasmids with inserts, by using the blue/white selection technique based on *lacZ*, which is well-known in selection procedures.

Thus for example the cloning sites might include BamHI, HindIII and/or EcoRI. Sites such as these are useful for sticky end cloning. Any suitable restriction site may be used, eg. according to choice or ease of construction etc.

The cloning region may further include additional restriction sites, for example C+G rich restriction sites such as NotI, EagI, XmaI, SmaI, BglI, and SfiI, for potential excision of the inserts. These may flank the cloning sites. Incidentally, such restriction sites, if desired, might also be used as cloning sites.

The cloning region may also further include a cos site for fosmid cloning, for example bacteriophage λ cosN. Appropriate sources and sequences are again well known in the art. Restriction sites, eg. Eco72I, may be included for blunt end fosmid cloning. In alternative embodiment, the cos site need not be located in the cloning region, and could be located elsewhere in the vector. More generally therefore, the cos site may be viewed as an optional feature of the vectors of the invention in general.

Combined fosmid and BAC vectors are a preferred feature of the present invention. Thus, the vector of the invention is preferably a BAC vector containing or comprising a cos site. Such a vector may be used for both BAC cloning eg. of larger or very large inserts and for cosmid cloning eg. of smaller inserts of 30-40kb.

The cloning region may further contain one or more bacteriophage P1 *loxP* sites for Cre-recombinase cleavage.

As mentioned above, the vector of the invention is no more than 15kb in size. The small size of the vector is advantageous in permitting cloning of large inserts. Preferably, the vector is no more than 14, 13 or 12kb in size.

A further advantageous feature is that the vector contains no more than 10%, preferably no more than 9, 8 or 7%, of the RK2 plasmid. Indeed, it is a surprising feature that a vector containing so little of RK2 is able to function as a broad host range plasmid for carrying large inserts.

In particular embodiments, the vector may contain only the *oriV, oriT* and *parDE* genes or genetic elements from RK2 and no other RK2-based or RK2-derived genes or genetic elements are included. In other embodiments, *parABC* may also be included. Further embodiments may include one or more antibiotic resistance markers from RK2 eg. tetracycline resistance (whether or not the vectors include *parABC* from RK2).

An advantageous feature of the vectors of the invention is that they may be used to clone inserts of large or very large size, as discussed above. As noted above an "insert" is the DNA to be cloned. It is accordingly a DNA molecule or fragment for cloning. It may be in any form suitable for cloning, eg. as a fragment with blunt or sticky ends. Inserts for introduction into the vectors may be obtained directly from a sample, eg. an environmental or other sample which may contain a source of DNA, typically a cell such a microorganism. Thus the DNA may be extracted or isolated by lysing the cells directly in the context of the sample. For example lysis buffer may be added directly to the sample. Alternatively, the cells (eg. microorganisms) may be isolated or separated from the sample before isolation of the DNA. This approach generally allows larger size DNA fragments to be isolated. The isolated DNA, or DNA for cloning obtained from other sources, may be size-fractionated to be prepare inserts for cloning. Further the isolated DNA or DNA fragments may be digested eg. partially digested using restriction enzymes or other means. Techniques for isolation of cells and DNA, digestion of DNA and size-fractionation or size-selection of DNA fragments are well-known to a person skilled in the art and widely described in the literature.

After appropriate inserts have been prepared they may be introduced or inserted into the vector. eg. by ligation into.a linearised and dephosphorylated vector. Again, procedures for this are standard in the art.

Although as noted above, the vector of the invention may be used to clone inserts of at least 12kb, it is preferred that the vector is capable of cloning large inserts of at least 30kb and particularly very large inserts of at least 80kb, as discussed above.

As noted above, an advantage of the present invention is that *oriV* allows the vector to be expressed at medium copy in the host cell, or higher. Whilst medium copy number is broadly defined herein to include copy number greater than 2, ie. 3 or more, it is preferred for the copy number (ie. the number of copies of the vector in a single cell or per host cell genome) to be 5 or more. Thus, more particularly a "medium" copy number may be 5 or more. Copy number may be increased to higher numbers, for example to high copy numbers of 10 or more. Thus, the copy number of the vector may be eg. 5-7 or 5-10, 5-12, 5-15, 5-20, 5-25 or even 7-20 or 10-20 or 7-25 or 10-25.

Replication from *oriV* requires the *trfA* gene, as explained above. In the vectors of the invention *trfA* is not present. Accordingly, for replication from *oriV* to occur the *trfA* gene must be separately provided. This is discussed further below, but a host cell may be engineered to express this gene, for example as in the EP1300^{™} *E. coli* cells available from Epicentre Technologies. Advantageously, the host may contain an inducible *trfA* gene. The *trfA* gene may either be on a separate vector (separate to the vector of the invention) or, more preferably, integrated into the host chromosome.

Modifications may be introduced into the *trfA* gene to increase copy number of the vector within a host cell as mentioned above, or to achieve temperature sensitive replication. Such modifications have been described in the literature. The copy number of RK2 within *E. coli* is usually estimated to be 5-7 plasmids per chromosome. However, this may be elevated in both *E. coli* and other bacteria by certain point mutation in the *trfA* gene, which may lead to copy numbers up to 23-fold higher than normal. Such "copy up" or *"cop* mutations" are described for example in Durland et al., 1990, J. Bacteriol, 172, 3859-3867; Haugan et al., 1992 supra; and Haugan et al., 1995, Plasmid, 33, 27-39. *Cop* mutations in the *trfA* gene may be used to increase expression in bacterial species beyond *E. coli.*

Studies have shown that cop mutations in *trfA* tend to be localised between the *Nde* I and *Sfi* I sites in *trfA,* and that cop mutations may readily be prepared by exchanging the *Sfi* I*lNde* I fragment internally in the *trfA* gene, and straight-forward, one-step cloning procedures (see Haugan et al., 1995, supra).

The vector of the invention may also contain other features or elements. Thus one or more selection markers may be present to facilitate the selection of transformants. Advantageously 2 or more selectable markers may be present. A wide range of selectable markers are known in the art and described in the literature. Any of these may be used according to the present invention and include for example the antibiotic resistance markers carried by the RK2 plasmids and their derivatives, or indeed any other plasmid. However, properties such as sugar utilisation, proteinase production or bacteriocin production or resistance may also be used as markers. The TOL plasmid *xylE* structural gene may also be used as a marker. This gene encodes the product C230 which may readily be detected qualitatively or assayed. Spraying a plate of bacterial colonies with catechol rapidly distinguishes C230⁺ colonies since they turn yellow due to the accumulation of 2-hydroxy muconic semialdehyde, enabling transformants/transconjugants etc. rapidly to be identified, by the presence of *xylE* in the vectors.

BAC, fosmid or other vectors used as starting or source vectors for construction of the vectors of the invention may already contain suitable selection markers eg. antibiotic resistance genes and these may be retained, or they may be supplemented or replaced. For example a BAC vector will typically contain the chloramphenicol resistance gene, Cm^{R} This may be supplemented by a further selection marker, eg. a further antibiotic resistance gene eg. the kanamycin resistance gene, Km^{R}.

The vector may further contain other features, for example to allow or facilitate study or analysis of the cloned insert. Thus the vector may contain primer binding sites for sequencing, eg. for BAC end sequencing.

The vector may further optionally contain sites flanking the cloning region that may be cut by very rare-cutting enzymes. Such enzymes are known to exist commercially. This may simplify interpretation of the insert fragments generated by standard enzymes as the vector part would always come out with a known band.

It may be desirable for the cloned insert to be expressed. Generally larger inserts may contain the necessary elements (eg. promoters, ribosome binding sites etc) for expression of genes within them. However, it may be desirable for the vectors of the invention to contain one or more expression control elements for expression of the inserts. Thus the vectors of the invention may further contain regulatory and/or enhancer functions for gene expression, for example transcriptional or translational control sequences such as start or stop codons, transcriptional initiators or terminators, promoters, ribosomal binding sites etc.

An advantage of the vectors of the invention is that are fully characterised, functionally and structurally. The vectors may thus be fully sequenced.

As mentioned above for replication of the vector from *oriV* to occur, the host must contain or be provided with the *trfA* gene. This may be provided to the host by means of a further vector, and preferably the *trfA* gene is inserted into the host chromosome. A further vector may be designed to achieve this.

Accordingly, the present invention further provides a vector system for cloning of DNA, said system comprising a first vector, being a cloning vector of the invention as defined above and a second vector comprising the *trfA* gene of RK2.

The second vector allows for expression of *trfA* in the host. Whilst this second vector may be eg. a plasmid or other vector which may be introduced into the host as an autonomously replicating element, it preferably allows for integration of the *trfA* gene into the host chromosome. Preferably the second vector carries a transposon. For example the *trfA* gene may be cloned into a suicide transposon vector. The second vector may contain expression control elements for expression of the *trfA* gene eg. transcriptional control elements such as a promoter. Advantageously, the trfA gene may be placed under the control of an inducible promoter eg. *Pm* or a mutant thereof. In this way expression of the gene can be controlled. The second vector may further contain a selection marker for selection of transformants.

Techniques for the preparation of such transposon vectors are well known in the art, as are techniques for introduction of the vectors into host cells eg. by electroporation

The cloning vector may be introduced into the host cell by standard transformation techniques, eg. heat-shock transformation. Methods for introducing cloning vectors into host cells and in particular methods of transformation of bacteria are well known in the art and widely described in the literature, including for example in Sambrook et al., (supra). Electroporation techniques are also well known and widely described.

The cloning vectors of the invention may be seen as based on the RK2 plasmid. A principle use of the vectors of the invention is in metagenomic cloning.

The cloning vector of the invention allows for the construction of metagenomic libraries in a broad range of hosts, wherein large inserts may be cloned at high copy number.

In the practice of the invention, DNA may be obtained and isolated from a desired source, eg. an environmental sample, as discussed above, DNA fragments for insertion into the cloning vector may be prepared and inserted using standard techniques. The cloning vectors thus prepared, ie. containing the inserts may then be introduced into a desired bacterial host cell eg. by transformation. The host cell for this step may be any desired host cell, and the vector may be introduced into one or more host cells eg. a range of desired hosts. The host may then be grown or cultured to create a library of cloned inserts. For example colonies of the transformed host cells may be plated. Preferably, the step of initial library construction is performed in a single host cell, following which the library is transferred to a range of other host cells by conjugative transfer ie using *oriT* of the vector. Such hosts for transfer are selected or modified to contain *trfA.*

The host cell for initial library contruction may be any desired host cell but conveniently will be *E. coli.* The second origin of the vector is selected to function in the host cell for initial library construction. Thus, the second origin may be an origin functional in *E. coli.* For example the second origin may conveniently be *ori2.* Advantageously, the *trfA* gene is not expressed in the first host to which the cloning vector is introduced (ie in which the library is initially constructed). Thus the first host may not contain *trfA* or if it is present, it.is not expressed eg. it is under the control of an inducible promoter which is not induced, or it is a temperature-sensitive mutant etc. Accordingly, in the first, or primary, host the vector replicates from the second origin and is accordingly present in a copy number of one or two only.

The library may then be transferred to the second or "secondary" hosts. One or more secondary hosts may be used, preferably, multiple hosts eg. 2 or more. preferably 3, 4, 5, 6, 8, 9 or 10 or more. This may be achieved by conjugative mating, using standard techniques. The mated secondary hosts may then be cultured. As noted above the secondary hosts contain *trfA.* Accordingly, in these hosts, the cloning vector may be replicated from *oriV* and hence at medium or high copy number. The present invention thus permits the gene dosage of the cloned inserts to be increased in a broad host range. Thus a library may first be created at low copy number, eg. in a primary host, and then transferred to a range of hosts and copy number increased.

Accordingly, in a further aspect the invention provides a method of cloning DNA said method comprising:
(i) introducing said DNA into a cloning vector as hereinbefore defined:
(ii) introducing said cloning vector into a first bacterial host cell, preferably into *E. coli:*
(iii) culturing said first host cell, thereby to clone said DNA;
(iv) transferring said cloned DNA into one or more secondary hosts.

More particularly, in said first host the vector replicates from said further (ie. the second) origin of replication and upon transfer to said secondary host, said vector replicates from *oriV.* Thus *trfA* is expressed in said secondary host but not in said first host.

Alternatively viewed, the invention provides a method of preparing a library of clones of DNA, said method comprising:
(i) introducing said DNA into a cloning vector as hereinbefore defined:
(ii) introducing said cloning vector into a first bacterial host cell, preferably into *E. coli*:
(iii) culturing said first host cell, thereby to prepare a first library of clones of said DNA;
(iv) transferring said first library into one or more secondary hosts to prepare one or more secondary libraries.

Mobilised *oriT*-mediated conjugation to a new host requires *tra* genes. If the first host cell does not contain these genes, it may be necessary to introduce them (eg into the first host cell prior to the first cloning step) or first to transfer the cloning vector from the first host into an intermediate host for subsequent transfer by conjugation. Thus, vectors from the first cloning step may be transformed into such an intermediate host. The step of transferring the cloned DNA/library may thus include step of introducing a vector containing said cloned DNA/library into an intermediate host, and transferring said cloned DNA/library from said intermediate host to said secondary host.

The invention will now be described in more detail in the following

Examples, with reference to the following drawings in which:

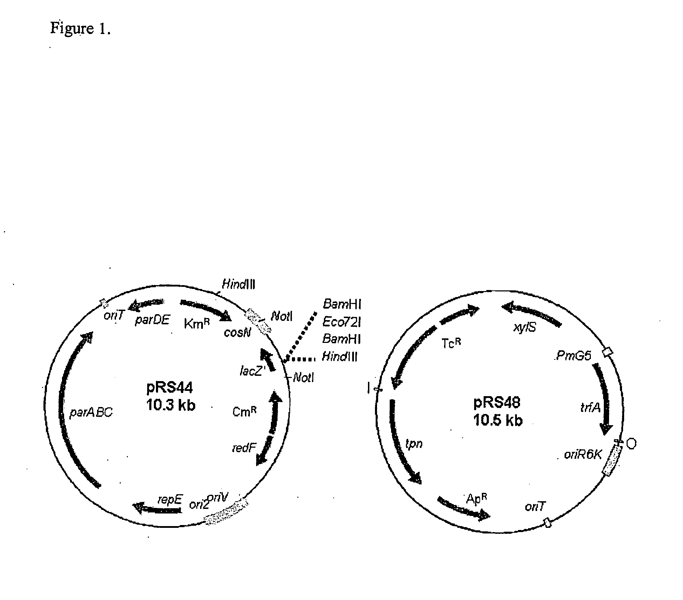

Figure 1 shows the plasmid vectors used in Example 1 for genomic library constructions (pRS44) and for support of vector replication in hosts other than *E. coli* (pRS48). pRS44 can replicate as a single copy replicon via *ori2* and *repE,* while *oriV* contributes to a medium copy-number if its replication initiation protein TrfA is expressed in the same cell. pRS44 DNA can easily be prepared in large quantities in the *E. coli* strain EPI300 by expressing a mutant *trfA* gene from an arabinose-induced promoter, as described by Wild *et al.* (Wild et al., 2002 Genome Res. 12: 1434-1444). *cosN* is the site used for packaging of the environmental DNA library in bacteriophage λ particles, *Bam*HI and *Eco72*I sites are used for BAC and fosmid cloning respectively and *Not*I is suitable for sizing of the inserts. The *trfA-*gene is inserted into the chromosome of hosts of interest by the transposon present in the narrow-host-range plasmid pRS48. The inside and the outside ends of the transposon (designated TnRS48) are marked I and O respectively. *tpn:* gene encoding the transposase, which is not a part of the transposon. *xylS*: gene encoding activator of *PmG5* transcription in the presence of benzoic acid type inducers, like m-toluate. *oriT*: origin of conjugative transfer. For further details see Table 1 and Examplel;.

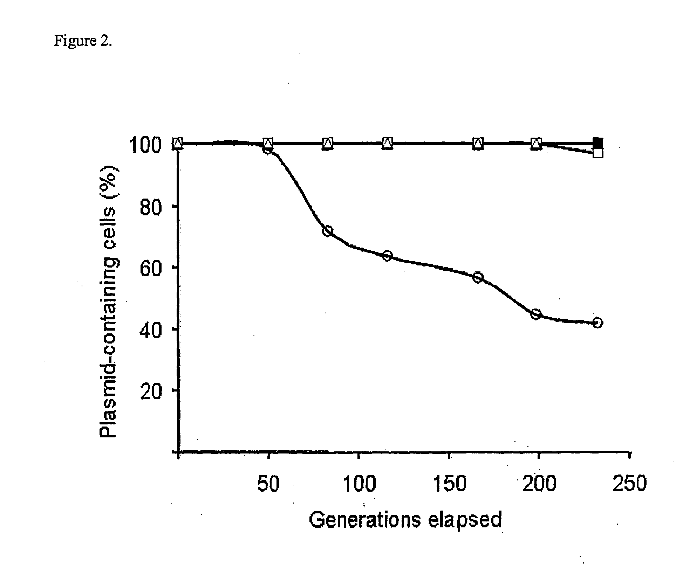

Figure 2 shows plasmid stability of pRS44 and pRS49 in the absence of antibiotic selection. Exponentially growing cells in shake flasks (in the presence of selection) were diluted 10⁵ times in medium lacking antibiotics. The cultures were then grown over-night and the dilution procedures were repeated until about 230 generations had elapsed. After each growth step cells were plated on L-agar lacking antibiotics. From each step 184 colonies were picked and duplicated into 96 well plates containing media with and without chloramphenicol. ■ pRS44, □ pRS49, △ RK2 and ○ pCC1FOS;

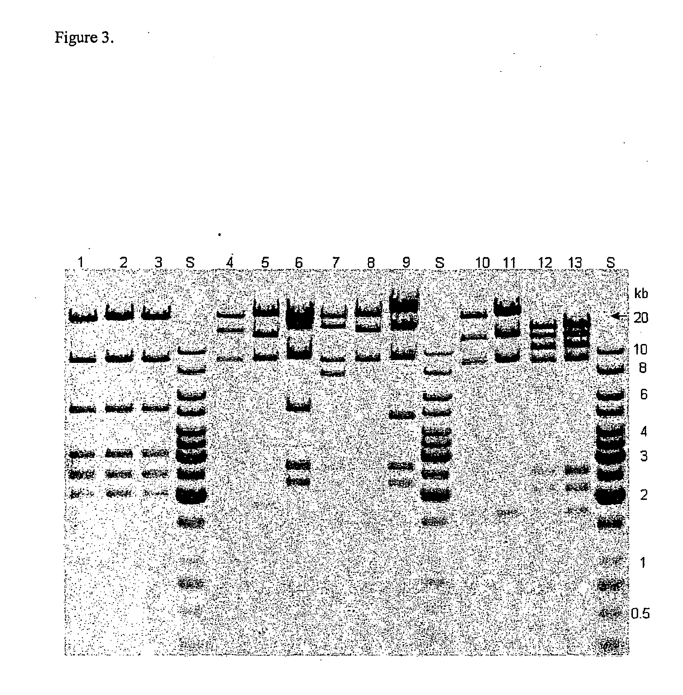

Figure 3 shows Agarose gel electrophoretic analysis of fosmid clones after passage through *P. fluorescens::*Tn*RS48* and *X. campestris::*Tn*RS48.* Lane 1. Plasmid 62 before transfer, and lanes 2 and 3 after transformation to *E. coli* from *P. fluorescens* and *X. campestris,* respectively. Lane 4: Plasmid 83 before transfer, and after transformation to *E. coli* from *P. fluorescens* (lanes 5-7) and *X. campestris* (lanes 8 and 9). Lane 10: Plasmid 37 before transfer, and after transformation to *E. coli* from *P. fluorescens.* (lanes 11 and 12) and *X. campestris* (lane 13). S: Molecular weight standard (Fermentas).

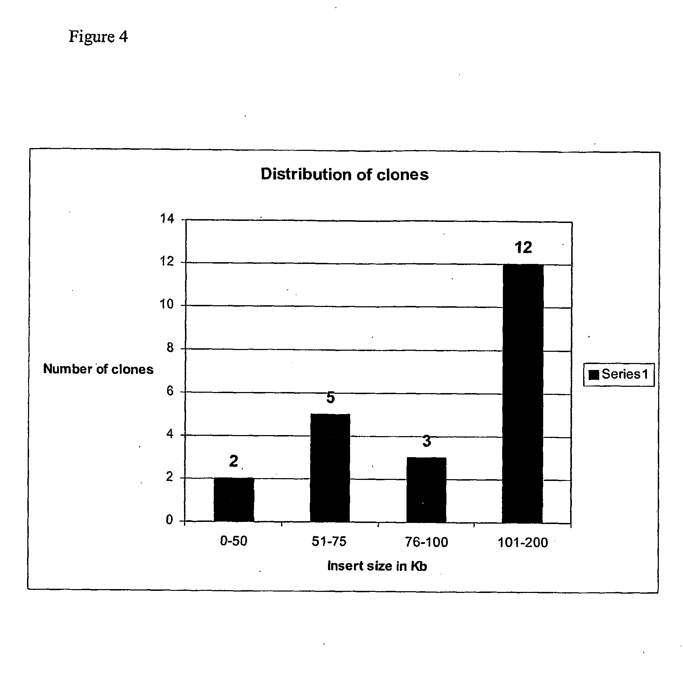

Figure 4 shows the insert size of prepared BAC vectors and the number of clones obtained with particular insert sizes.

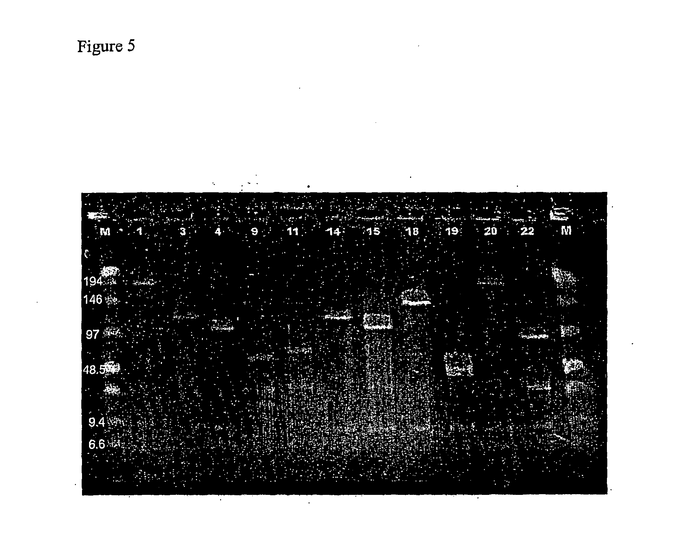

Figure 5 shows agarose gel electrophoretic analysis of 11 of the obtained plasmids digested with Not-1. The lane numbers shown represent those inserts described under "Size test results" described in Example 2. M: Molecular weight standard (New England Biolabs).

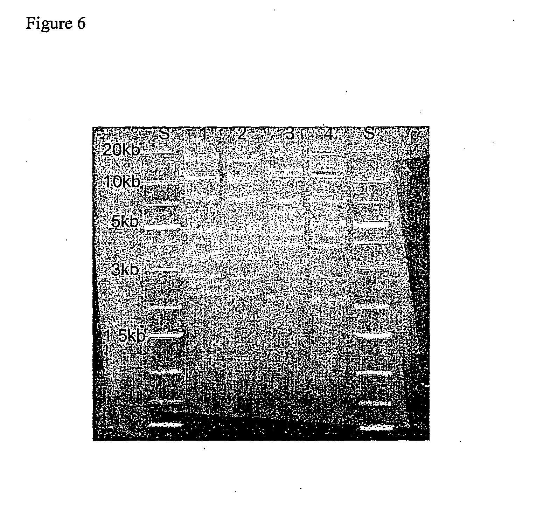

Figure 6 shows agarose gel electrophoretic analysis of HindIII digested BAC clones before and after passage through *P.fluorescens*:: TnRS48. Lane 1 and 2: Plasmid B9 before and after transfer; respectively. Lane 3 and 4: Plasmid B19 before and after transfer, respectively. S: Molecular weight standard (Fermentas).

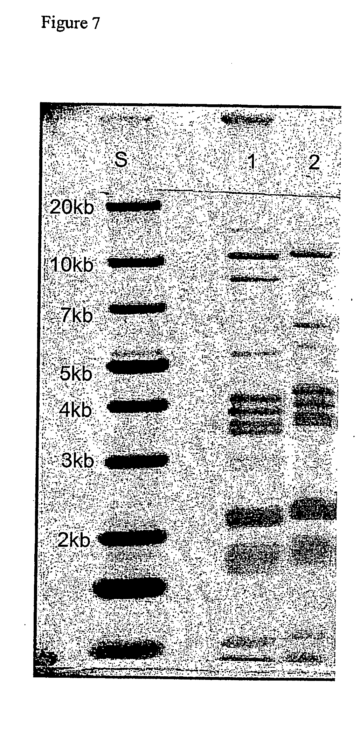

Figure 7 shows Southern blot analysis of HindIII digested plasmid BIO (Lane 1) and total DNA from a BIO *P. fluorescens* transconjugant (Lane 2). The entire plasmid isolated from *E. coli* was labelled and used as probe against total DNA isolated from *P. fluorescens.* S: Molecular weight standard (Fermentas).

### Example 1

### Materials and methods

### Bacterial strains, plasmids, and growth media

The bacterial strains and plasmids used in this study are described in Table 1. *E. coli* strains were grown in Luria-Bertani (LB) medium or on L-agar at 37°C. *Pseudomonas fluorescens* and *Xanthomonas campestris* strains were grown at 30°C in LB or on Difco PIA agar (*P. fluorescens*) and in YM broth or on YM agar (*X. campestris*). Antibiotics (when relevant) were used at the following concentrations: chloramphenicol, 12.5 µg/ml (*E. coli*), 30 µg/ml (*X. campestris*); kanamycin, 50 µg/ml (*E. coli* and *X campestris*); tetracycline 10 µg/ml (*E. coli*), 15 µg/ml (*X. campestris*) or 25 µg/ml (*P. fluorescens*). 5-bromo-4-chloro-3-indoyl-β-D-galactopyranoside (X-gal) and isopropyl-β-D-thiogalactoside (IPTG) were used for blue-white selection of clones with inserts in *E. coli.* Clones in EPI300 were switched from single copy- to high copy-number by L-arabinose induction, using the solution from the Copy Control Fosmid Library Production Kit, Epicentre. Expression of *trfA* from *PmG5* was induced by addition of m-toluate at 0.5 mM.

### Standard DNA manipulations

Agarose gel electrophoresis, and routine DNA manipulations were performed according to the methods of Sambrook and Russel, 2001 in Molecular cloning, a laboratory manual. 3rd ed. Cold Spring Harbor Laboratory Press, Cold Spring Harbor, NY, or by using commercially available kits. DNA sequencing was performed using the Big-Dye Terminator v1.1 Cycle kit (Applied Biosystems) or by MWG-Biotech AG.

### Vector constructions, transformation and conjugative matings

A broad-host-range vector (pRS44) for fosmid and BAC cloning was constructed by introducing the *parDEoriT* region and a kanamycin resistance gene to pCCIFOS. The entire nucleotide sequence of this vector is known. The suicide transposon vector pRS48 was constructed by cloning *trfA* and the tetracycline resistance gene into pKD20 (Bakkevig et al., JBacteriol 187: 8375-8384), using *E. coli* S17.1(λ*pir*) (De Lorenzo et al., 1993 J Bacteriol 175: 6902-6907) as host. For further details, see Table 1.

The transposon in pRS48 was inserted into the chromosomes of electrocompetent *P. fluorescens* NCIMB10525 and *X. campestris* B100-152 (Hötte et al., 1990 J Bacteriol 172: 2804-2807) by standard electroporation (13 V/cm, 100 ohm, 25 µF), as described for *E. coli* by Sambrook and Russel (Sambrook & Russel, 2001), and the transformants designated NCIMB10525::Tn*RS48* and B100-152::Tn*RS48* were selected on PIA or YM agar containing tetracycline. pRS44-derived clones were transformed into *E. coli* S 17.1 (Simon et al., 1983 Bio/Technology 1: 784-79) by standard heat-shock transformation (Chung et al., 1989 Proc Natl Acad Sci USA 86: 2172-2175).

Conjugative matings were performed on L-agar without antibiotic selection over-night at 30°C. The mixtures were then plated on PIA with kanamycin (*P. fluorescens*) or on YM-agar with tetracycline and chloramphenicol (*X. campestris*), followed by incubation at 30°C for 48 and 72 hours, respectively.

Plasmids were isolated from cultures of *P. fluorescens* and *X. campestris* by Wizard Plus SV Minipreps (Promega) and electroporated into EPI300 (13 V/cm, 100 ohm, 25 µF).

### Sample collection and DNA extraction

Water from the surface micro layer at six different locations along the Trondheims fjord (Norway) was collected during the period July to October 2004, essentially as described by Garrett (Garret, 1965 Limnol and Oceanogr 10: 602-605). The samples, each of 20-25 litres, were first pre-filtered though a 250 µm grid to remove the largest particles, then filtered through nylon filters with 60 µm pore size (Millipore). The filtrates were concentrated in two steps, first by tangential flow filtration using a Pellicon XL50 ultra filtration system, followed by further concentration in an Amicon stirred cell (Millipore). The samples were then diluted in 2 x STE-buffer (1 M NaCl, 0.1 M Na₂EDTA, 10 mM Tris-HCl, pH 8.0) to give a final DNA concentration of 150-250 µg/ml. The cell suspensions were mixed with an equal volume of 2% InCert agarose (Cambrex) in PBS-buffer (0.8% NaCl, 0.02% KCI, 0.144%Na₂HPO₄, 0.024 % KH₂PO₄, pH 7.4) and agarose plugs were molded in disposable plug molds (BioRad).

High molecular weight DNA was extracted according to the method of Stein *et al.* (Stein et al., Archaeon. J Bacteriol 178: 591-599), but with some modifications. The agarose plugs were lysed in 25 ml lysis buffer (10 mM Tris-HCl, 50 mM NaCl, 0.1 mM Na₂EDTA, 1% N-Lauroyl Sarcosine sodium salt, 0.2% sodium deoxycholate, 1 mg/ml lysozyme, pH 8.0) for 3 hours. The plugs were transferred to 25 ml EPS buffer (1% N-Lauroyl Sarcosine sodium salt and 1 mg/ml proteinase K in 0.5 M Na₂EDTA, pH 8) and incubated at 55°C for 16 hours. Proteinase K was inactivated and the plugs were dialysed and stored as described by Osoegawa et al. (1999 Construction of bacterial artificial chromosome (BAC/PAC) libraries. Current Protocols in Human Genetics, (Dracopoli NC, Haines JL, Korf BR, Morton CC, Seidman CE, Seidman JG & Smith DR, eds), unit 5.15, Wiley, New York).

### Construction of gene libraries

The small insert library was made by randomly cloning partially *Sau*3AI-digested environmental DNA into the *Bam*HI site of the multi copy plasmid vector pLitmus28. The inserts were partially sequenced and aligned using BLAST algorithms. The fosmid library was constructed essentially according to the procedures described in the Copy Control Fosmid Library Production kit protocol, Epicentre.

### Results and Discussion

### Construction of a broad-host-range metagenome vector system

A broad-host-range fosmid and BAC-vector (pRS44, Fig. 1) was constructed using the commercially available pCCIFOS vector as a starting point. pCC1FOS has two origins of replication, the F-factor origin (*ori2*) and *oriV* from RK2. *ori2* functions in *E. coli* and is active during construction of libraries in this host, while it is not active in most other hosts. *oriV* can be activated by expressing the replication initiation protein TrfA in the host of interest. The strategy we have used here is to introduce the origin of conjugative transfer (*oriT*) to pCC1FOS to allow conjugation to non-*E. coli* hosts. We have also inserted the stabilization element *parDE* from RK2 to reduce the chances that the recombinant plasmids with large inserts are lost from the new hosts. Finally, the kanamycin resistance gene was inserted to provide an alternative selection marker, potentially useful in some hosts. The *Bam*HI site is useful for sticky end BAC-cloning, while the *Eco*72I site is used for blunt end fosmid cloning. The *lac* system for blue-white screening was kept from pCC1FOS.

For easy integration of *trfA* in non-*E. coli* hosts, we constructed a suicide vector (pRS48, Fig. 1) which facilitates insertion of a derivative of transposon Tn5 expressing the TrfA protein. This protein is expressed from the inducible *PmG5* promoter, a mutant derivative of *Pm,* which is known to be active in many hosts (Mermod et al., 1986 J Bacteriol 167: 447-454; Ramos et al., 1988 FEBS Lett 226: 241-246; Keil & Keil, 1992 Plasmid 27: 191-199). The inducibility in addition allows for modification of the amount of TrfA produced. pRS48 replicates in the *E. coli* strain S17.1λ(pir) which expresses the Pir protein, needed for replication initiation of the plasmid R6K origin, *oriR6K* (De Lorenzo *et al.,* 1993 supra).

### Plasmid stability in the absence of selection

Plasmid stability may potentially become critical for the functioning of the metagenome cloning vector described here, and to quantify this we measured the rate by which it became lost in the absence of antibiotic selection in *E. coli* EPI300 (Fig. 2). As controls in this experiment we used the native RK2 plasmid and pCC1FOS. By the use of repeated transfers growth was monitored over about 230 generations, a number which enormously exceeds the number of generations taking place in laboratory scale batch cultures. The experiments showed that plasmid loss could easily be detected for pCC1FOS, while both pRS44 and a derivative of it containing a 36 kb control DNA insert (pRS49) were remarkably stable, like whole RK2. This experiment therefore clearly confirmed the relevance of introducing *parDE* into the vector.

### Construction of a metagenomic library in pRS44

Initial experiments showed that the standard 36 kb insert used as a control for the commercially available vectors were packaged and established in *E. coli* at similar frequencies for pRS44 as for pCC1FOS. However, we wanted to test the vectors with DNA from the environment, because it is well known that it may be difficult or inefficient to clone DNA from such samples and because we wanted to test the behaviour of the environmental DNA inserts in new hosts. In our laboratory we have a running project involving studies of microorganisms from sea surface layers, which have been demonstrated to contain a much higher concentration of organic matter and bacteria than the underlying water. We therefore decided to use such a DNA sample to test the vector system.

To make sure that the available DNA samples actually originated from environmental bacteria we first constructed a small insert library in *E. coli* using the standard high copy-number cloning vector pLitmus28. Ninety-one clones from this library were sequenced from one of the insert ends and subsequently analysed by BLAST homology searches. This analysis showed that 18.7% of the sequences displayed no significant hits to other sequences present in the database (E-value>1), 32.9% had E-values between e⁻¹⁰ and 1, and for 48.4% of the hits the E-value was < e⁻¹⁰. Ten of the best BLAST-hits are shown in Table 2, and from these data one can see that the hits are all against bacteria, several of which are typically found in marine environments (DeLong, 2005 Nature Rev 3: 459- 469). These results are therefore consistent with the assumption that the DNA sample mainly originates from marine bacteria, a conclusion that is not necessarily as trivial as it may seem (DeLong, 2005, supra).

The DNA from the sea surface microlayer was then used to construct a small fosmid library (about 400 clones) in pRS44. Restriction digest analysis of 16 of these clones indicated that the insert sizes varied in a range from about 20 to 35 kb. None of the restriction patterns were the same, showing that the clones were not siblings (data not shown). This small library was therefore sufficient to test the concept of transfer to hosts other than *E. coli.*

### Transfer of plasmids containing environmental DNA from E. coli to P. fluorescens and X. campestris

The *E. coli* strain EPI300 does not contain the *tra* genes required for mobilized *oriT*-mediated conjugation to new hosts, and for this reason selected plasmids from the library were first transformed into strain S17.1, which has the RK2 *tra* genes integrated into the chromosome. We have found that it is easy to obtain large numbers of transformants using the library as source DNA, so this additional step will not represent a limitation for later transfer of plasmids from large libraries to new hosts.

Before plasmids from the library were transferred to two selected hosts, *P. fluorescens* and *X. campestris,* the transposon in pRS48 (carrying the *trfA* gene) was inserted into their chromosomes by electroporation. The library plasmids could then be conjugatively transferred to and replicate in these hosts. Kanamycin (*P. fluorescens*) and chloramphenicol (*X. campestris*) resistant clones were obtained at high frequencies, demonstrating that very large libraries could easily have been transferred. Plasmids were isolated from independent transconjugants, retransformed into *E. coli* EPI300, digested with HindIII and analysed by agarose gel electrophoresis (Fig. 3).

Lanes 1, 2 and 3 show that the digests were identical for a randomly selected plasmid designated 62, before and after passage through the two non-*E*. *coli* hosts. However, more such studies clearly showed that this case represents an oversimplification of what happens in general. Lanes 5, 6 and 7 show that the band patterns obtained after passage through *P. fluorescens* are not the same for another plasmid (designated 83), even though they all originate from the same *E. coli* clone in the library. Lanes 8 and 9 show the digests of plasmid 83 transformed from *X. campestris* back to *E. coli.* The plasmid in lane 8 appears identical to that of plasmid 83 before transfer, but the plasmid in lane 9 has clearly been changed during or after transfer. Interestingly, inspection of the digestion patterns showed that in all cases the structural modifications involved increases in plasmid sizes.

The fact that clone 83 could be retained both in its original and in a modified form from the same host (lanes 8 and 9) was considered puzzling, and a third example illustrates another rather surprising observation. Lane 10 shows the restriction fragment pattern of a plasmid designated 37 before transfer to *P. fluorescens* and *X. campestris.* After transfer and retransformation back to *E. coli* several different patterns were observed, and three selected examples are shown in lanes 11-13. The band pattern of the plasmid in lane 11 (from *P. fluorescens*) could not be distinguished from that before transfer (lane 10), while those in lanes 12 (from *P. fluorescens*) and 13 (from *X. campestris*) are clearly different. Plasmid sizes had again increased, but more interestingly, the modifications appear to be the same in spite of the fact that the plasmids had passed through two different species.

### Implications of the results for use of minimal RK2 replicons in metagenome research

The experiments reported here show that the broad-host-range vector is very stably maintained even if it contains large inserts, and it can be transferred to presumably almost any Gram-negative host. We have also done preliminary attempts to test the ability of pRS44 to contain inserts of sizes larger than those limited by λ packing. These experiments have so far shown that we could stably maintain pRS44 derivatives of at least 80 kb in size in *E. coli* (data not shown), indicating that the RK2 replicon system may be used for construction of metagenome libraries with insert sizes typical for established narrow-host-range BAC vectors. The results presented here show that the plasmids seem to be quite frequently changed during passage through new hosts. Interestingly, modifications of plasmid structure have also been previously observed in conjugation experiments with strain S17.1 (Priefer et al., 1985 J Bacteriol 163: 324-330). Since the modifications do not happen in all transfers for any specific clone, the problem can be overcome by simply screening a larger number of transconjugants than would otherwise be required.

In all cases tested so far the modifications of the plasmids involved insertions (increase in total size), and interestingly, we have recently found by DNA sequencing that the additional DNA always originated from the donor *E. coli* strain (data not shown). This means that the problem most likely is not related to the recipient bacteria, but is the result of events taking place in the donor during the conjugation process. Based on this assumption it also appears easier to understand how the plasmids could be modified in the same way after transfer through two different species (Fig. 3, lanes 12 and 13). Thus, by investigating how these modifications take place in *E. coli* it should be possible to eliminate the problem. Such experiments are therefore now ongoing in our laboratory.

**Table 1: Bacterial strains and plasmids used in this study**

| Bacterial strain or plasmid | Properties^{a} | Source or Reference |
|---|---|---|
| *E. coli* | | |
| EPI300 | Phage T1-resistant and *lacZ* strain with L-arabinose induced chromosomally expressed TrfA, ( F- *mcrA* Δ*(mrr-hsdRMS-mcrBC)* ϕ*80dlacZ*Δ*M15* Δ*lacX74 recA1 endA1 araD139* Δ*(ara, leu)7697 galU galK* λ⁻ *rpsL nupG trfA tonA dhrf)* | Epicentre |
| S17.1 | Strain with the RK2 *tra* genes for conjugative transfer integrated in the chromosome *(RP4-2-Tc::Mu-Km::Tn7, pro, res⁻ mod⁺,* Tp^{r} Sm^{r}) | Hötte *et al.,* 1990 |
| S17.1*(*λ*.pir)* | λ*pir* lysogen of strain S 17.1 | De Lorenzo *et al.,* 1993 |
| *Psedomonas. fluorescens* | | |
| NCIMB 10525 | *P. fluorescens* wild type | NCIMB |
| NCIMB 10525::Tn*RS48* | Derivative of NCIMB 10525 with transposon Tn*RS48* from pRS48 integrated into the chromosome | This work |
| *Xanthomonas. campestris* | | |
| B100-152 | Spontaneous *xanA* exopolysaccharide-negative mutant | Simon *et al.,* 1983 |
| B100-152::Tn*RS48* | Derivative of B100-152 with transposon Tn*RS48* from pRS48 integrated into the chromosome | This work |
| Plasmids | | |
| pCC1FOS | Cloning vector for Copy Control Fosmid library containing *ori2* and *oriV, parABC, cos* and *loxP,* Cm^{r}, 8.1 kb | Epicentre |
| pHH100G5 | RK2 based plasmid containing a mutant *Pm* promoter designated *PmG5,* Km^{r}, 8.8 kb | Gimmestad *et al.,* 2004 |
| pJB321 | RK2 minimal replicon containing *parDE,* Ap^{r}, 5.6 kb | Blatny *et al.,* 1997a |
| pJB658 | RK2 expression vector containing the *Pm* promoter and the gene encoding the regulatory protein XylS and TrfA, Ap^{r}, 6.8 kb | Blatny *et al.,* 1997b |
| pKD20 | Suicide vector encoding a mini-Tn5 transposon with *Nde*I*lNot*I cloning sites for introduction of genes under *PmG5*/*xylS* control, Ap^{r}, Km^{r}, 8.0 kb | Bakkevig *et al.,* 2005 |
| pLitmus28 | *ColE1* replicon, Ap^{r}, 2,8 kb | New England Biolabs |
| pLitmusTcBam2 | Litmus28 derivative containing *tetA* and *tetR,* Ap^{r}, Tc^{r}, 5.1 kb | Bakkevig *et al.,* 2005 |
| pRS43 | Derivative of pCCIFOS with an insertion of a 1.2 kb PCR fragment encoding *parDEoriT* from pJB321, Cm^{r}, 9.3 kb | This work |
| pRS44 | Derivative of pRS43 with an insertion of a 1.0 kb PCR fragment encoding Km^{r} from pHH100G5, Cm^{r}, Km^{r}, 10.3 kb | This work |
| pRS49 | pRS44 with Fosmid Control DNA (Epicentre) cloned in the Eco27I-site, Cm^{r}, Km^{r}, 46.3 kb | This work |
| pRS47 | *trfA* from pJB658 cloned as a 1.2 kb *Nde*I/*Not*I PCR fragment into pKD20, Km^{r}, 9.1 kb | This work |
| pRS48 | Derivative of pRS47with a 2.2 kb *Bam*HI fragment containing *tetA* and *tetR* from pLitmusTcBam2 cloned in the *Dra*ITI/*SacI* sites, Tc^{r}, 10.5 kb | This work |
| RK2 | Ap^{r}. Km^{r}, Tc^{f}, 60.1 kb | Pansegrau *et al.,* 1994 |

| | | |
|---|---|---|
| ^{a} Ap^{r}: ampicillin resistance; Cm^{r}: chloramphenicol resistance; Km^{r}: kanamycin resistance; Tc^{r}, tetracycline resistance | | |

Bakkevig K, Sletta H, Gimmestad M, Aune R, Ertesvåg H, Degnes K, Christensen BE, Ellingsen TE & Valla S (2005) Role of the Pseudomonas fluorescens alginate lyase (AlgL) in clearing the periplasm of alginates not exported to the extracellular environment. J Bacteriol 187: 8375-83384.

Blatny JM, Brautaset T, Winther-Larsen HC, Haugan K & Valla S (1997a) Construction and use of a versatile set of broad-host-range cloning and expression vectors based on the RK2 replicon. Appl Environ Microbiol 63: 370-379.

Blatny JM, Brautaset T, Winther-Larsen HC, Karunakaran P & Valla S (1997b) Improved broad-host-range RK2 vectors useful for high and low regulated gene expression levels in Gram-negative bacteria. Plasmid 38: 35-51.

De Lorenzo V, Cases I, Herrero M & Timmis KN (1993) Early and late responses of TOL promoters to pathway inducers: identification of postexponential promoters in Pseudomonas putida with lacZ-tet bicistronic reporters. J Bacteriol 175: 6902-6907.

Gimmestad M, Sletta H, Karunakaran P, Bakkevig K, Ertesvåg H, Ellingsen TE, Skjåk-Bræk G & Valla S (2004) Patent WO 2004/011628 A1, New mutant strains of *Pseudomonas fluorescens* and variant thereof, methods for their production, and uses thereof in alginate production.

Hötte B, Rath-Arnold I, Pühler A & Simon R (1990) Cloning and analysis of a 35.3-kilobase DNA region involved in exopolysaccharide production in Xanthomonas campestris pv. campetris. J Bacteriol 172: 2804-2807.

Pansegrau W, Lanka E, Barth PT, Figurski DH, Guiney DG, Haas D, Helinski DR, Shwab H, Stanisich VA & Thomas CM (1994) Complete nucleotide sequence of Birmingham IncPα plasmids - compilation and comparative analysis. J Mol Biol 239: 623-663.

Simon R, Priefer U & PübJer A (1983) A broad host range mobilization system for in vivo genetic engineering: transposon mutagenesis in Gram negative bacteria. BiolTechnology 1: 784-791

**Table 2: The best BLAST hits for 10 of the end-sequenced inserts of the small insert library.**

| Best BLAST hit | E-value | Enzyme | Acc.# |
|---|---|---|---|
| *Shewanella oneidensis* | 1e⁻¹⁰³ | Hypothetical amidohydrolase | NP_715659 |
| *Vibrio parahaemolyticus* | 6e⁻⁹³ | Putative cation efflux system transmembrane protein | NP_799990 |
| *Vibrio vulnificus* | 3e⁻⁹⁰ | Geranylgeranyl pyrophosphate synthase | AAO08844 |
| *Vibrio vulnificus* | 2e⁻⁸¹ | Predicted amino acid racemase | AAO07615 |
| *Rhodopirellula baltica* | 9e⁻⁷³ | Conserved hypothetical protein-putative eukaryotic thiol proteases | NP_870899 |
| *Vibrio vulnificus* | 3e⁻⁶⁷ | Autotransporter adhesin | AAO08378 |
| *Photorhabdus luminescens* | 5e⁻⁶⁴ | Acetyl-CoA carboxylase alpha subunit | CAE12983 |
| *Photobacterium profundum* | 1e⁻⁶¹ | Putative aspartate carbamoyltransferase | CAG18904 |
| *Escherichia coli* | 2e⁻⁵⁵ | PmbA protein | NP_757180 |
| *Pseudomonas aeruginosa* | 3e⁻⁵² | Putative aldolase | NP_252120 |

### Example 2

### Materials and methods

### Bacterial strains, plasmids, and growth media

The bacterial strains and plasmids used in this study are described in Table 3.

**Table 3: Bacterial strains and plasmids used in this study.**

| Bacterial strain or plasmid | Properties^{a} | Source or Reference |
|---|---|---|
| *E. coli* | | |
| EPI300 | Phage T1-resistant and *lacZ*⁻ strain with L-arabinose induced chromosomally expressed TrfA, ( F⁻ *mcrA* Δ*(mrr-hsdRMS-mcrBC)* ϕ*80dlacZ*Δ*M15* Δ*acX74 recA1 endA1 araDl39*Δ*(ara, leu)7697 galU galK* λ⁻ | Epicentre |
| | r*psL nupG trfA tonA dhrf*) | |
| S17.1 | Strain with the RK2 *tra* genes for conjugative transfer integrated in the chromosome *(RP4-2-Tc::Mu-Km::Tn7, pro, res⁻ mod⁺,* Tp^{r} Sm^{r}) | Simon *et al.,* 1983 |
| *Pseudomonas.* | | |
| *fluorescens* | | |
| NCIMB 10525::Tn*RS* | Derivative of NCIMB 10525 with transposon TnRS48 | This work |
| *48* | from pRS48 integrated into the chromosome | |
| Plasmids | | |
| pCC1FOS | Cloning vector for Copy Control Fosmid library containing *ori2* and *oriV, parABC, cos* and *loxP,* cm^{r}, 8.1 kb | Epicentre |
| pHH100G5 | RK2 based plasmid containing a mutant Pm promoter designated *PmG5,* Km^{r}, 8.8 kb | Gimmestad et *al., 2004* |
| pJB321 | RK2 minimal replicon containing *parDE,* Ap^{r}, 5.6 kb | Blatny *et al.,* 1997a |
| pJB658 | RK2 expression vector containing the *Pm* promoter and the gene encoding the regulatory protein XylS and TrfA, Ap^{r}, 6.8 kb | Blatny *et al.,* 1997b |
| pKD20 | Suicide vector encoding a mini-Tn5 transposon with *Nde*I*lNot*I cloning sites for introduction of genes under *PmG5*/*xylS* control, Ap^{r}, Km^{r}, 8.0 kb | Bakkevig *et al.,* 2005 |
| pLitmus28 | *ColE1* replicon, Ap^{r}, 2,8 kb | New England Biolabs |
| pLitmusTcBam2 | Litmus28 derivative containing *tetA* and *tetR,* Ap^{r}, Tc^{r}, 5.1 kb | Bakkevig *et al.,* 2005 |
| pRS43 | Derivative of pCCIFOS with an insertion of a 1.2 kb PCR fragment encoding *parDEoriT* from pJB321, Cm^{r}, 9.3 kb , | This work |
| pRS44 | Derivative of pRS43 with an insertion of a 1.0 kb PCR | This work |
| | fragment encoding Km^{r} from pHH100G5, Cm^{r}, Km^{r}_{.} 10.3 kb | |
| pRS47 | *trfA* from pJB658 cloned as a 1.2 kb *Nde*I*lNot*I PCR fragment into pKD20, Km^{r}, 9.1 kb | This work |
| pRS48 | Derivative of pRS4 7with a 2.2 kb *Bana*HI fragment containing *tetA* and *tetR* from pLitmusTcBam2 cloned in the *Dra*III/*Sac*I sites, Tc^{r}, 10.5 kb | This work |
| pTA44 | Derivative of pRS44 in which a HindIII site is removed by site specific mutagenesis, t→c in position 727 | This work |
| RK2 | Ap^{r}, Km^{r}, Tc^{r}, 60.1 kb | Pansegrau *et al.,* 1994 |

| | | |
|---|---|---|
| ^{a} Ap^{r}: ampicillin resistance; Cm^{r}: chloramphenicol resistance; Km^{r}: kanamycin resistance; Tc^{r}, tetracycline resistance References are as indicated for Table 1. | | |

*E. coli* strains were grown in Luria-Bertani (LB) medium or on L-agar at 37°C. *Pseudomonas fluorescens* was grown at 30°C in LB or on Difco PIA agar. Antibiotics (when relevant) were used at the following concentrations: chloramphenicol, 12.5 µg/ml (*E. coli*), kanamycin, 50 µg/ml (*E. coli* and *P. fluorescens*); tetracycline 10 µg/ml (*E. coli*) or 25 µg/ml (*P. fluorescens*). Expression of *trfA* from *PmG5* was induced by addition of m-toluate at 0.5 mM.

### Standard DNA manipulations

Agarose gel electrophoresis and routine DNA manipulations were performed according to the methods of Sambrook and Russel (2001, Molecular cloning, a laboratory manual. 3rd ed. Cold Spring Harbor Laboratory Press, Cold Spring Harbor, NY) or by using commercial available kits.

### Site specific mutagenesis to remove a HindIII site from pRS44

Site specific mutagenesis was performed using the QuickChange Site-Directed Mutagenesis Kit from Stratagene on the plasmid pHH100G5. pHH100G5 carries the kanamycin resistance gene that was originally incorporated into the pRS43 vector to give pRS44. The mutagenised kanamycin resistance gene was then cut out from this plasmid and ligated into pRS43 to give pTA44.

### BAC construction

BAC clones with inserts up to around 200 kb were constructed by Bio S&T Inc. (Canada). High-molecular-weight DNA from the nuclei of the plant *Ipomoea nil* was used as cloning material, and the vector pTA44 was used as a BAC cloning vector as described below.

### Prepar-ation and partial digestion of high-molecular-weight DNA

The method for high-molecular-weight (HMW) DNA preparation from plant nuclei of *Ipornoea nil* is described by Zhang, H.B., X.P. Zhao, A.H. Patterson, and R.A. Wing. 1995. The Plant Journal 7:175-184.

Nuclei were prepared from 100 grams of leaves and embedded in 5ml of 2% (w/v) low-melting-point agarose plugs. Partial digestion of the plugs was performed with 10 units of Hind III per plug for 10 minutes at 37°C. Reactions were stopped by adding 1/10 volume of ice cold 0.5M EDTA (pH8.0).

### Size separation, isolation of size-selected DNA by electro-elution

Partially digested HMW DNA was size-selected on a 1% (w/v) pulsed field agarose gels in 0.5X TBE on a CHEF DRIII (Bio-Rad, Canada). Size selection was performed by PFGE for 12 hours at 11°C with a constant pulse time of 90s and 6V/cm. The gel slice containing 50-350kb was subjected to electro-elution by PFGE for 5 hours with a constant pulse time of 30s and 6V/cm. The eluted DNA fragments were dialysized against 1x TE (10mMTris-HCl, 1mM EDTA, pH8.0) buffer for at least 2 hours before ligation.

### BAC Construction Vector preparation

pTA44 was used. Following transformation into DH10B, a Maxiprep kit (Qiagen, Canada) was used for vector DNA purification. Purified vector DNA was subjected to HindIII digestion and dephosphorylation, followed by phenol/chloroform purification (standard protocol). Purified digested DNA was dissolved at 25ng/µl.

### Ligation

80-100ng partially-digested size-selected DNA fragments were ligated to 20ng of vector DNA (pTa44-HindIII and pIndigoBAC-HindIII) in a volume of 50µl with 1X ligase buffer and 3 units of ligase (USB, Canada) at 14°C for overnight incubation.

### Transformation and clone characterization

ElectroMax DH10B from Invitrogen was used as a host strain for transformation. Two microliters of ligation mix were used to transform 20µl of *E*. *coli* cells. Cells were recovered by shaking at 100rpm for 1 hour at 37°C in 600µl of SOC medium (Invitrogen, USA). 60µl of the transformed cells were then selected on LB medium supplemented with chloramphenicol (12.5mg/L), X-GAL and IPTG, by incubation at 37°C overnight. The number of white clones was recorded and about 20 clones were handpicked and cultured for a miniprep. The miniprep was carried out using the following steps:
- Spin down an overnight 5ml culture (500g for 5 minutes).
- Remove supernatant.
- Add 200µl of P1 solution from Qiagen.
- Add 200µl of P2 solution from Qiagen.
- Incubate 5 minutes at room temperature.
- Add 200µl of ice-cold P3 solution from Qiagen.
- Incubate on ice for 10 minutes.
- Spin down (13,000g) for 15 minutes.
- Transfer supernatant to a new tube and add 1 volume of isopropanol (about 420µl; about 70% of transferred volume (600µl).
- Place at -20 degrees for 1 hour.
- Spin down (13,000g) for 20 minutes.
- Remove supernatant.
- Add 70% ethanol (420µl).
- Spin down (13,000g) for 5 minutes.
- Allow to air dry.
- Resuspend in TE or water (add about 50µl).

Minipreped DNA was digested with NotI and then subjected to a PFGE gel for characterization.

### Size test results

The estimated size of the clones are (in kb) as shown in Figure 4:

| | | | | | |
|---|---|---|---|---|---|
| 1. | 195 | 9. | 130 | 17. | 80 |
| 2. | 48 | 10. | 190 | 18. | 150 |
| 3. | 135 | 11. | 110 | 19. | 100 |
| 4. | 120 | 12 | 70 | 20. | 195 |
| 5. | 58 | 13 | 73 | 21. | 83 |
| 6. | 75 | 14. | 135 | 22. | 131 |
| 7. | 70 | 15. | 120 | | |
| 8. | 48 | 16. | 95 | | |

As a comparison, the average size of 20 clones for pIndigoBAC was calculated to be about 100kb, which is similar to that of pTA44.

### Ligation and transformation efficiency

The efficiency was determined by the number of white clones obtained on a selective medium per 1/10 volume (i.e. 60ul) following transformation (Table 4).

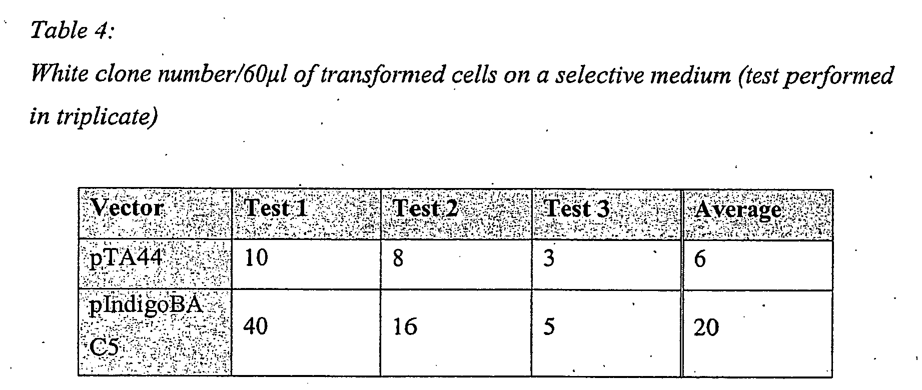

The discrepancy among tests may be attributed to the unstable and lower quality of a batch of DH10B cells obtained from Invitrogen.

### Transfer of BAC clones to Pseudomonas fluorescens

Selected BAC clones were conjugated from the *E. coli* strain S17.1 to *Pseudomonas fluorescen::*Tn*RS48* Conjugative matings were performed on L-agar without antibiotic selection over-night at 30°C. The mixtures were then plated on PIA with kanamycin and m-toluate, followed by incubation at 30°C for 48 hours.

Plasmids were isolated from cultures of *P. fluorescens* by the miniprep method described above and electroporated into EPI300 (or 517.1).

### Results

### Construction of pTA44

The presence of two recognition sites for the restriction enzyme HindIII in the cloning vector pRS44 (one in the cloning site and one in the kanamycin resistance gene) makes BAC cloning with HindIII digested DNA impossible for this vector. Therefore, site specific mutagenesis was performed to remove the HindIII site in the kanamycin resistance gene, thus creating vector pTA44.

### Construction of BAC clones

By using high-molecular-weight DNA from plant nuclei and the cloning vector pTA44, BAC clones with inserts up to around 200 kb were obtained. Pulse field gel electrophoresis was used to determine the size of the inserts, and Figure 5 show the result for 22 of the obtained BAC clones. The ligation and transformation efficiency was similar to that observed in a parallel experiment with the commercially available BAC cloning vector, plndigoBAC5 (Epicentre).

### Transfer of BAC clones to Pseudomonas fluorescens

Six selected BAC clones with insert sizes from 100kb - 195 kb (Table 5) were transferred to one tested alternative species, *Pseudomonas fluorescens* through conjugation. All the selected BAC clones gave transconjugants in *P. fluorescens::*Tn*RS48.*

**Table 5: Names and insert sizes of the six BAC clones that were transferred to P. fluorescens. Here, B1 represents the same plasmid as the one labelled "1" in Figure 5 and under the heading "Size test results", B9 = 9, etc.**

| BAC clone: | B1 | B9 | B10 | B18 | B19 | B20 |
|---|---|---|---|---|---|---|
| Insert size [kb]: | 195 | 130 | 190 | 150 | 100 | 195 |

### Stability of transferred BAC clones

The stability of three of the transferred plasmids was investigated either by southern analysis of restriction digested total DNA from *P. fluorescens* transconjugants (B10), or by retransferring plasmids from *P. fluor-escens* transconjugants to *E. coli,* with following restriction analysis(B9 and B19).

From the restriction pattern it seems that plasmids B9 and B19 could be retransferred in an intact form (Figure 6), demonstrating that plasmids with inserts up to at least 130 kb are stable in both species.

Southern blot analysis showed that plasmid with an insert size of 190 kb can be maintained in *P. fluorescens* (Figure 7). In this case we were unable to get *E. coli* transformants back (technical problem), but the Southern blot analysis shows that the plasmid is there. There are some band differences between the *E. coli* plasmid and the corresponding pattern from *P. fluorescens,* and this is most likely a result of the problems observed for the conjugation process (see Example 1), and that it has nothing to do with plasmid stability in *P. fluorescens.* This demonstrates that the constructed vectors (pRS44/pTA44) can maintain very large inserts in more than one (probably numerous) species.

## Claims

1. A cloning vector for cloning of DNA in a broad host range of bacteria, said vector being an autonomously replicating artificial chromosome comprising:
(i) the RK2 origin of replication *oriV*;
(ii) the RK2 origin of conjugative transfer *oriT*;
(iii) par DE from RK2
(iv) a cloning region
(v) a further origin of replication which permits replication of said vector at a copy number of no more than 1 or 2, wherein preferably the further origin is *ori2* or the P1 origin of replication;
wherein said vector is no more than 15kb in size, does not contain *trfA* of RK2, and is capable of cloning inserts of at least 12kb, preferably at least 30kb, or preferably at least 80kb, and wherein the content of RK2 DNA in said vector is no more than 10% of RK2.

2. The cloning vector of claim 1 wherein said DNA is metagenomic DNA or DNA from an environmental sample.

3. The cloning vector of claim 1 or 2 wherein said vector is an artificial chromosome.

4. The cloning vector of claim 3 wherein said artificial chromosome is a bacterial artificial chromosome (BAC) or a P1-derived artificial chromosome (PAC).

5. The cloning vector of any one of claims 1 to 4 wherein said vector comprises *ori2, repE* and *parAB.*

6. The cloning vector of claim 5 wherein said vector comprises *parC* and/or *redF.*

7. The cloning vector of any one of claims 1 to 6 wherein said vector comprises a P1 plasmid replicon, optionally a P1 packaging site (pac) and optionally two P1 *lox P* recombination sites.

8. The cloning vector of any one of claims 1 to 7, further comprising a cos site, wherein preferably the cloning vector is a combined fosmid and BAC vector.

9. The cloning vector of any one of claims 1 to 8 wherein said vector comprises one or more selectable markers.

10. A vector system for cloning of DNA said system comprising the cloning vector of any one of claims 1 to 9 and a second vector comprising the *trf A* gene of RK2, wherein preferably said second vector comprises a transposon.

11. The vector system of claim 10 wherein said *trfA* gene is under the control of an inducible promoter, preferably Pm or a mutant thereof.

12. A host cell containing the cloning vector of any one of claims 1 to 9.

13. Use of the vector of any one of claims 1 to 9 for metagenomic cloning.

14. A method of cloning DNA, said method comprising;
(i) introducing said DNA into a cloning vector of any one of claims 1 to 9
(ii) introducing said cloning vector into a first bacterial host cell;
(iii) culturing said first host cell, thereby to clone said DNA;
(iv) transferring said cloned DNA into one or more secondary hosts, wherein preferably replication in said first host cell occurs from the further origin of replication (v) and wherein replication in said one or more secondary hosts occurs from *oriV,* and/or wherein preferably said first host cell is *E.coli.*

15. A method of preparing a library of clones of DNA, said method comprising;
(i) introducing said DNA into a cloning vector or any one of claim 1 to 9;
(ii) introducing said cloning vector into a first bacterial host cell;
(iii) culturing said first host cell, to prepare a first library of clones of said DNA;
(iv) transferring said first library into one or more secondary hosts to prepare one or more secondary libraries.

## Patentansprüche

1. Klonierungsvektor zum Klonieren von DNA in einem breiten Wirtsbereich von Bakterien, wobei der Vektor ein autonom replizierendes künstliches Chromosom ist, das enthält:
(i) den RK2-Replikationsursprurig *oriV*;
(ii) den RK2-Ursprung des konjugativen Transfers *oriT;*
(iii) par DE von RK2
(iv) einen Klonierungsbereich
(v) einen weiteren Replikationsursprung, der die Replikation des Vektors bei einer Kopienzahl von nicht mehr als 1 oder 2 ermöglicht, wobei der weitere Ursprung vorzugsweise *ori2* oder der P1-Replikationsursprung ist;
wobei der Vektor nicht mehr als 15 kb groß ist, kein *trf A* von RK2 enthält und in der Lage ist, Einschübe von mindestens 12 kb, vorzugsweise mindestens 30 kb oder vorzugsweise mindestens 80 kb zu klonieren, und wobei der Gehalt an RK2-DNA in dem Vektor nicht mehr als 10 % RK2 ist.

2. Klonierungsvektor nach Anspruch 1, wobei die DNA metagenomische DNA oder DNA von einer Umgebungsprobe ist.

3. Klonierungsvektor nach Anspruch 1 oder 2, wobei der Vektor ein künstliches Chromosom ist.

4. Klonierungsvektor nach Anspruch 3, wobei das künstliche Chromosom ein bakterielles künstliches Chromosom (BAC) oder ein von P1 abgeleitetes künstliches Chromosom (PAC) ist.

5. Klonierungsvektor nach einem der Ansprüche 1 bis 4, wobei der Vektor *ori2, repE* und *parAB* aufweist.

6. Klonierungsvektor nach Anspruch 5, wobei der Vektor parC und/oder redF aufweist.

7. Klonierungsvektor nach einem der Ansprüche 1 bis 6, wobei der Vektor ein P1-Plasmid-Replikon, wahlweise eine P1-Verpackungsstelle (pac) und wahlweise zwei P1 *lox P* Rekombinationsstellen aufweist.

8. Klonierungsvektor nach einem der Ansprüche 1 bis 7, der weiterhin eine cos-Stelle aufweist, wobei der Klonierungsvektor vorzugsweise ein kombinierter Fosmid-und BAC-Vektor ist.

9. Klonierungsvektor nach einem der Ansprüche 1 bis 8, wobei der Vektor einen oder mehrere auswählbare Marker aufweist.

10. Vektorsystem zum Klonieren von DNA, wobei das System den Klonierungsvektor nach einem der Ansprüche 1 bis 9 und einen zweiten Vektor mit dem trfA-Gen von RK2 aufweist, wobei der zweite Vektor vorzugsweise ein Transposon aufweist.

11. Vektorsystem nach Anspruch 10, wobei das trfA-Gen unter der Steuerung eines induzierbaren Promotors, vorzugsweise Pm oder einer Mutante davon, steht.

12. Wirtszelle, die den Klonierungsvektor nach einem der Ansprüche 1 bis 9 enthält.

13. Verwendung des Vektors nach einem der Ansprüche 1 bis 9 zum metagenomischen Klonieren.

14. Verfahren zum Klonieren von DNA, wobei das Verfahren umfasst:
(i) Einführen der DNA in einen Klonierungsvektor nach einem der Ansprüche 1 bis 9
(ii) Einführen des Klonierungsvektors in eine erste bakterielle Wirtszelle;
(iii) Kultivieren der ersten Wirtszelle, um **dadurch** die DNA zu klonieren;
(iv) Übertragen der klonierten DNA in einen oder mehrere sekundäre Wirte,
wobei die Replikation in der ersten Wirtszelle vorzugsweise vom weiteren Replikationsursprung (v) stattfindet bzw. ausgeht und wobei die Replikation in dem einen oder den mehreren sekundären Wirten von *oriV* stattfindet bzw. ausgeht und/oder wobei vorzugsweise die erste Wirtszelle *E.coli* ist.

15. Verfahren zum Erstellen einer Bibliothek von Klonen von DNA, wobei das Verfahren umfasst:
(i) Einführen der DNA in einen Klonierungsvektor nach einem der Ansprüche 1 bis 9;
(ii) Einführen des Klonierungsvektors in eine erste bakterielle Wirtszelle;
(iii) Kultivieren der ersten Wirtszelle, um eine erste Bibliothek von Klonen der DNA zu erstellen;
(iv) Übertragen der ersten Bibliothek in einen oder mehrere sekundäre Wirte, um eine oder mehrere sekundäre Bibliotheken zu erstellten.

## Revendications

1. Vecteur de clonage pour le clonage de l'ADN dans un large spectre d'hôtes bactériens, ledit vecteur étant un chromosome artificiel se répliquant de manière autonome comportant :
(i) l'origine de réplication RK2 *oriV* ;
(ii) l'origine de transfert conjugatif RK2 *oriT* ;
(iii) le système parDE de RK2 ;
(iv) une région de clonage ;
(v) une autre origine de réplication qui permet la réplication dudit vecteur en un nombre de copies non supérieur à 1 ou 2, dans lequel, de préférence, l'autre origine est *ori2* ou l'origine de réplication P1 ;
dans lequel ledit vecteur n'est pas supérieur à 15 kb en taille, ne contient pas le gène *trfA* de RK2 et peut cloner des inserts d'au moins 12 kb, de préférence d'au moins 30 kb, ou de préférence d'au moins 80 kb, et dans lequel le contenu d'ADN de RK2 dans ledit vecteur n'est pas supérieur à 10 % de RK2.

2. Vecteur de clonage selon la revendication 1, dans lequel l'ADN est l'ADN métagénomique ou l'ADN issu d'un échantillon environnemental.

3. Vecteur de clonage selon la revendication 1 ou 2, dans lequel ledit vecteur est un chromosome artificiel.

4. Vecteur de clonage selon la revendication 3, dans lequel ledit chromosome artificiel est un chromosome artificiel bactérien (BAC) ou un chromosome artificiel dérivé de P1 (PAC).

5. Vecteur de clonage selon l'une quelconque des revendications 1 à 4, dans lequel ledit vecteur comporte *ori2, repE* et *parAB.*

6. Vecteur de clonage selon la revendication 5, dans lequel ledit vecteur comporte *parC* et/ou *redF.*

7. Vecteur de clonage selon l'une quelconque des revendications 1 à 6, dans lequel ledit vecteur comporte un réplicon de plasmide P1, facultativement un site d'encapsidation de P1 (pac) et facultativement deux sites de recombinaison de P1 *loxP.*

8. Vecteur de clonage selon l'une quelconque des revendications 1 à 7, comportant en outre un site cos, dans lequel, de préférence, le vecteur de clonage est un fosmide et un vecteur BAC combinés.

9. Vecteur de clonage selon l'une quelconque des revendications 1 à 8, dans lequel ledit vecteur comporte un ou plusieurs marqueurs sélectionnables.

10. Système de vecteur pour le clonage de l'ADN, ledit système comportant le vecteur de clonage selon l'une quelconque des revendications 1 à 9 et un second vecteur comportant le gène *trfA* de RK2, dans lequel, de préférence, ledit second vecteur comporte un transposon.

11. Système de vecteur selon la revendication 10, dans lequel ledit gène *trfA* est sous le contrôle d'un promoteur inductible, de préférence Pm ou un mutant de celui-ci.

12. Cellule hôte contenant le vecteur de clonage selon l'une quelconque des revendications 1 à 9.

13. Utilisation du vecteur de l'une quelconque des revendications 1 à 9 pour le clonage métagénomique.

14. Procédé de clonage de l'ADN, ledit procédé comportant les étapes consistant à :
(i) introduire ledit ADN dans un vecteur de clonage de l'une quelconque des revendications 1 à 9 ;
(ii) introduire ledit vecteur de clonage dans une première cellule hôte bactérienne ;
(iii) mettre en culture ladite première cellule hôte pour ainsi cloner ledit ADN ;
(iv) transférer ledit ADN cloné dans un ou plusieurs hôtes secondaires,
dans lequel, de préférence, une réplication dans ladite première cellule hôte se produit à partir de l'autre origine de réplication (v) et dans lequel une réplication dans ladite ou lesdites cellules hôtes secondaires se produit à partir de *oriV,* et/ou dans lequel, de préférence, la première cellule hôte est *E.coli.*

15. Procédé de préparation d'une banque de clones de l'ADN, ledit procédé comportant les étapes consistant à :
(i) introduire ledit ADN dans un vecteur de clonage de l'une quelconque des revendications 1 à 9 ;
(ii) introduire ledit vecteur de clonage dans une première cellule hôte bactérienne ;
(iii) mettre en culture ladite première cellule hôte, pour préparer une première banque de clones dudit ADN ;
(iv) transférer ladite première banque dans un ou plusieurs hôtes secondaires pour préparer une ou plusieurs banques secondaires.
